# EUROPEAN PATENT SPECIFICATION

(11) **EP 1 648 483 B1**
(45) Date of publication and mention of the grant of the patent: **04.06.2008**
(21) Application number: 04740472.8
(22) Date of filing: 30.06.2004
(51) Int. Cl.: A61K 36/185, A61K 36/23, A61K 36/752, A61K 36/48, A23L 1/0524, A61P 3/10, A61P 3/06, A23L 1/0526, A23L 1/053

(54) **COMPOSITION FOR TREATING AND/OR PREVENTING INSULIN RESISTANCE**
ZUSAMMENSETZUNG ZUR BEHANDLUNG UND/ODER VORBEUGUNG VON INSULINRESISTENZ
COMPOSITION DE TRAITEMENT ET/OU DE PREVENTION DE LA RESISTANCE A L'INSULINE

(30) Priority: 30.06.2003 EP 03014816
(43) Date of publication of application: 26.04.2006
(73) Proprietor: Nestec S.A., 1800 Vevey (CH)
(72) Inventor: POUTEAU, Etienne, CH-1005 Lausanne (CH); KREMPF, Michel, FR-44230 Saint Sebastien/Loire (FR); MACE, Catherine, CH-1005 Lausanne (CH)
(74) Representative: Dixon, Sarah
(86) International application number: PCT/EP2004/007092
(87) International publication number: WO 2005/002612

(56) References cited:
- DE-A- 10 117 185
- US-A- 4 496 606
- US-A1- 2002 019 334
- US-A1- 2003 004 095
- US-A1- 2003 008 810
- LIBRENTI M C ET AL: "EFFECT OF SOYA AND CELLULOSE FIBERS ON POSTPRANDIAL GLYCEMIC RESPONSE IN TYPE II DIABETIC PATIENTS" DIABETES CARE, vol. 15, no. 1, 1992, pages 111-113, XP009019275 ISSN: 0149-5992
- BOMMARTINI F ET AL: "GUAR-CAROB BEAN ASSOCIATION IN THE CONTROL OF POST-PRANDIAL HYPERGLYCEMIA" GIORNALE DI GERONTOLOGIA, vol. 33, no. 6, 1985, pages 497-504, XP009019278 ISSN: 0017-0305
- FELDMAN NIRA ET AL: "Enrichment of an Israeli ethnic food with fibres and their effects on the glycaemic and insulinaemic responses in subjects with non-insulin-dependent diabetes mellitus" BRITISH JOURNAL OF NUTRITION, vol. 74, no. 5, 1995, pages 681-688, XP009019279 ISSN: 0007-1145
- ANDERSON JAMES W ET AL: "Effects of psyllium on glucose and serum lipid responses in men with type 2 diabetes and hypercholesterolemia" AMERICAN JOURNAL OF CLINICAL NUTRITION, vol. 70, no. 4, October 1999 (1999-10), pages 466-473, XP002260585 ISSN: 0002-9165
- TUOMILEHTO J ET AL: "A double-blind evaluation of guar gum in patients with dyslipidaemia." HUMAN NUTRITION. CLINICAL NUTRITION. MAR 1983, vol. 37, no. 2, March 1983 (1983-03), pages 109-116, XP009035434 ISSN: 0263-8290
- REID ROBERT ET AL: "Dietary counselling for dyslipidemia in primary care: results of a randomized trial." CANADIAN JOURNAL OF DIETETIC PRACTICE AND RESEARCH : A PUBLICATION OF DIETITIANS OF CANADA = REVUE CANADIENNE DE LA PRATIQUE ET DE LA RECHERCHE EN DIETETIQUE : UNE PUBLICATION DES DIETETISTES DU CANADA. 2002 WINTER, vol. 63, no. 4, January 2002 (2002-01), pages 169-175, XP001183223 ISSN: 1486-3847
- DATABASE MEDLINE [Online] US NATIONAL LIBRARY OF MEDICINE (NLM), BETHESDA, MD, US; 15 December 1987 (1987-12-15), CASIMIRRI F ET AL: "[Effect of guar on plasma lipids and on the biological activity of insulin in patients with type 2 diabetes mellitus]" XP002293526 Database accession no. NLM2827061 & MINERVA MEDICA. 15 DEC 1987, vol. 78, no. 23, 15 December 1987 (1987-12-15), pages 1753-1757, ISSN: 0026-4806

## Description

The present invention relates to the use of a composition comprising acetogenic fibres for the preparation of a nutritional and/or a pharmaceutical composition for treating and/or preventing insulin resistance and to nutritional or pharmaceutical compositions and functional food products containing these ingredients.

Diabetes mellitus and insulin resistance both are metabolic disorders exhibiting a major common manifestation, hyperglycaemia.

Diabetes mellitus originates from an inherited and/or acquired deficiency in the production of insulin by the pancreas, and/or by the ineffectiveness of the insulin produced, hepatic and peripheral tissues becoming resistant to insulin action. Such a deficiency in insulin secretion and insulin sensitivity eventually results in increased concentrations of glucose in the blood, which in turn damage many of the body's systems, in particular the blood vessels and nerves.

There are two principle forms of diabetes, Type 1 and Type 2.

In Type 1 diabetes the pancreas of affected individuals fails to produce insulin largely due to a destruction of the islets of Langerhans, which in most cases seem to occur as a consequence of an auto-immune reaction triggered by some environmental factor, such as a viral infection. Heavy lymphocytic infiltrates appear in and around islets with the number and size of islets being reduced, eventually leading to decreased insulin production and glucose intolerance. This form develops most frequently in children and adolescents, but is being increasingly noted later in life.

Type 2 diabetes results from the body's inability to properly respond to the action of insulin produced by the pancreas. It occurs most frequently in adults, but is being noted increasingly in adolescents as well. The islets of Langerhans are normal in number or somewhat reduced with type II diabetes mellitus. Fibrosis and deposition of amylin polypeptide within islets are most characteristic of the chronic states of Type 2 diabetes.

Diabetes mellitus of both types is associated with a number of life-threatening and/or handicapping diseases. Examples are nodular and diffuse glomerulosclerosis, which may lead to chronic renal failure. Diabetics are prone to infections, particularly pyelonephritis. Also the eyes may be affected with diabetic retinopathy being one of the leading causes for irreversible blindness. Most persons with Type 1 diabetes and many of those with Type 2 diabetes develop some sort of background (non-proliferative) retinopathy. In severe cases, neo-vascularization may lead to adhesions (synechiae) between iris and cornea or iris and lens, eventually leading to secondary glaucoma with blindness. Also cataracts are more common in diabetics. This predilection for development of cataracts is felt to result from hyperglycaemia leading to accumulation of sorbitol that results in osmotic damage to the crystalline lens.

Persons with diabetes mellitus, either Type 1 or Type 2, also exhibit early and accelerated atherosclerosis. The most serious complications of this are atherosclerotic heart disease, cerebrovascular disease, and renal disease, with the most common cause of death being myocardial infarction. Peripheral vascular disease is a particular problem with diabetes mellitus and is made worse through the development of diabetic neuropathy, leading to propensity for injury. Mucormycosis is another feared complication in individuals experiencing diabetes mellitus. The site of involvement is typically the nasopharyngeal region, but the infection can spread to involve soft tissues and bone of the face, orbit, skull, and brain.

The treatment of individuals suffering from diabetes generally involves physical activity, diet and/or administration of medicaments. People with Type 1 diabetes are usually totally dependent on insulin injections for survival, requiring daily administration. Type 2 diabetic patients usually have to observe a strict diet and may additionally receive oral anti-diabetics, such as sulphonyl ureas, alpha-glucosidase inhibitors and biguanides, or even insulin injections, the administration of which is often associated with severe side effects and complications.

The majority of people suffer from Type 2 diabetes, which accounts for around 90% of all diabetes cases world-wide. On the molecular level Type 2 diabetes is characterized by a defect of both insulin secretion and action. The defect of insulin secretion relates mostly to the first phase of the post-prandial insulin release from pancreas, wherein in diabetic patients the already formed insulin is stored within the β-cells, but cannot be released into circulation. Indeed, most of the Type 2 diabetic patients present a resistance to the action of the insulin such that in order to cope with similar glucose concentration as present in healthy people, Type 2 diabetics require a higher concentration of insulin in plasma.

Another type of abnormality in glucose metabolism is insulin resistance, that is, a reduced sensitivity in the tissues of the body to the action of insulin, which goes along with a perturbed lipid (blood fats) metabolism, obesity, and high blood pressure. This cluster of abnormalities has come to be known as a syndrome, going by a variety of names, including Syndrome X, the Deadly Quartet, and the Insulin Resistance Syndrome.

When insulin resistance, or reduced insulin sensitivity, exists, the body attempts to overcome this resistance by secreting more insulin from the pancreas. The development of Type 2, or non-insulin dependent, diabetes occurs when the pancreas fails to sustain this increased insulin secretion. The importance of the Insulin Resistance Syndrome, or perhaps more accurately, "The Pluri-Metabolic Syndrome", lies in its consequences. The syndrome is typically characterized by varying degrees of glucose intolerance, abnormal cholesterol and/or triglyceride levels, high blood pressure, and upper body obesity, all independent risk factors for cardiac disease.

Following a meal, a person suffering insulin resistance will have elevated glucose circulating in the blood, signalling yet more insulin to be released from the pancreas until the glucose is taken up by the cells. Experts suggest that 11 to 25 percent of the adult population may be resistant to insulin to some degree.

Due to the increasing number of affected people world-wide and the changing lifestyle of the society there exists a need in the art to provide additional means useful in preventing, treating and/or improving conditions associated with Type 2 diabetes mellitus and/or insulin resistance. Moreover, such a means should be essentially free from disadvantageous side-effects well known from many oral anti-diabetics, and should be easy to take up.

It is known that supplementation of food with dietary fibres may be helpful in preventing or treating a large variety of gastro-intestinal disorders, such as constipation, intestinal toxemia, cholethiasis, colon cancer, and colitis, etc. and may positively influence lipid metabolism by interfering with cholesterol absorption, changing lipoprotein lipase activity or fatty acid metabolism. Some of the positive effects generally associated with a fibre supplementation of food have been associated with the formation of short chain fatty acids (SCFAs: acetate, propionate and butyrate) as products of bacterial fermentation of fibres in the gut. Among these SCFAs, acetate is often the major product and is known to be readily absorbed by the colonic mucosa, and it has been shown that acetate can supply 6 to 10% of the basal energy expenditure in humans. In particular, acetate may be activated into acetyl-CoA and later involved in free fatty acid synthesis for the building of epithelial membranes or may enter mitochondria yielding ketone bodies and providing energy.

Surprisingly, the present inventors have now found that acetogenic fibres have significant effects in improving insulin sensitivity, and in particular, in re-establishing normal insulin-sensitivity and thus a normal systemic metabolism.

The present invention therefore provides the use of a composition comprising acetogenic fibres for the preparation of a nutritional and/or a pharmaceutical composition for treating, preventing and/or improving insulin resistance.

Compositions comprising fibers such as soy fibers have been used to treat diabetic patients due to their ability to delay the absorption of glucose from the gut into the bloodstream (see for example Librenti et al Diabetes Care, Vol. 15, No. 1, 1992, pp 111-113).

The term "dietary fibre" is generally understood to designate non-starch polysaccharides which cannot be digested by human enzymes and which pass intact through the stomach and small intestine arriving unchanged at the large intestine. In the large intestine, these fibres are fermented by the intestinal bacteria to produce gases, short chain fatty acids and esters of such acids, principally acetates, propionates and butyrates. The term "acetogenic fibre" is used herein to designate those dietary fibres which, upon fermentation in the large intestine produce predominantly acetic acid and acetates. Dietary fibres are generally classified in this way in the literature, however, they may be fermented in vitro by batch techniques devised to simulate the conditions devised in the large intestine and the relative amounts of acetate, propionate and butyrate may be measured. When measured by this technique, an acetogenic fibre may be considered to be a fibre which, when fermented, produces at least 60% acetic acid/acetates. An alternative measure is the amount of acetate produced in which case an acetogenic fibre may be considered to be a fibre which, when fermented, produces at least about 600 µmoL of acetate per 100 mg of fibres in 24- hours in *in vitro* conditions with human inoculums. Examples of such fibres include lactulose, pectins such as citrus pectin, apple pectin, and carrot pectin, gum Arabic, soybean fibre, soy fibre and acacia gum and mixtures of these fibres may also be used, for example 20% apple pectin with 80% acacia gum. Soluble or low-viscous fibres, that is, non-gel forming fibres having a low viscosity in aqueous solutions are preferred.

The acetogenic fiber may be incorporated in the present composition in an amount of from about 0.2 to 90 % by weight, preferably from 0.5 to 70 % by weight, more preferably 0.7 to 30 % by weight, even more preferably 5 to 25 % by weight, most preferred about 7% by weight, based on the total weight of the composition.

Without wishing to be bound to any theory it is presently assumed that an increased amount of acetate in blood and tissues - resulting from an administration of a composition according to the present invention results in reduced lipolysis, i.e. a reduced liberation of glycerol and fatty acids from tissues into the blood. This could result in a reduction in the amount of free fatty acids inactivating insulin receptors, which, in turn, could result in an improvement in insulin sensitivity even to the levels present in healthy persons.

Compositions according to the invention will also be of high interest for large parts of the population, which are not suffering from insulin resistance or Type 2 diabetes mellitus at present, but belong to a target group at risk to develop any of said disorders, either due to a high risk diet or genetic predisposition. Moreover, an increase in insulin sensitivity is also highly interesting for other groups of persons, such as patients recovering from diseases or trauma leading to muscle depletion, exercising persons or elderly persons, since insulin is an anabolic hormone necessary for muscle mass maintenance and growth.

The composition as described above may of course also be used for the manufacture of a so called functional food product or a pharmaceutical composition.

During the first administrations of the composition according to the invention, one has to keep in mind that the acetogenic fibres have to be digested in the colon; therefore, it is preferable that the composition is absorbed between 3 and 7 hours before a meal, for example 4 hours. After a few administrations of the composition, we observe an increased insulin sensitivity, and in that second phase the composition may be consumed either together with a meal, in particular a meal containing carbohydrates, or shortly before or after such a meal, such as up to half an hour, or preferably, up to 10 minutes before or after such a meal. The composition may be taken separately or as a supplement to a meal.

Particularly good results may be achieved when providing at least 0.1 g of acetogenic fibers per kg body weight, more preferably between 0.1 to 1.5 g of acetogenic fibers per kg body weight, most preferably between 0.3 to 0.8 g of acetogenic fibers per kg body weight, even more preferably 0.5 g of acetogenic fibers per kg body weight, e.g. during, before or after a standard meal, in particular a standard meal comprising carbohydrates. A standard meal is any meal comprising at least 150 kcal, more preferably at least 250 kcal.

The nutritional composition according to the present invention is preferably enterally administrable, such as in form of a powder, a liquid concentrate, or a ready-to-drink beverage. The composition can be directly consumed or admixed with various foodstuffs, in particular ready-to-use snacks such as biscuits or bars, dairy products or drinks, or used for the preparation of an oral or enteral nutritional composition or a fruit juice.

A composition according to the present invention may of course comprise other conventional ingredients, such as proteins, digestible carbohydrates, lipids, vitamins and minerals, other fibres both soluble and insoluble, food additives etc..

In particular, vitamins and minerals may be present in an amount of between 30 % and 150 % of US RDA (US recommended (daily) dietary allowance) per daily dosage. Additionally, one or more food grade emulsifiers may be included in the nutritional composition, if desired, such as diacetyl tartaric acid esters of mono- and diglycerides, lecithin, and mono- or diglycerides or a mixture thereof. Similarly, suitable food-acceptable salts and/or stabilizers may also be included.

If a protein source is included, it preferably comprises preferably 21 to 40 % by weight, more preferably about 25 to 35 % by weight of the composition. Suitable protein sources include whey proteins such as sweet whey, pea proteins and soy proteins.

If a lipid source is included, it preferably comprises about 5% to 40 % of the energy (measured in calories) on the basis of the total energy of the composition; preferably, about 10 % to about 20 % of the energy. Any suitable fat or fat mixture may be used. Vegetable fat is particularly suitable, for example soy oil, palm oil, coconut oil, safflower oil, sunflower oil, corn oil, canola oil, lecithin and the like. Animal fat such as milk fat may also be added if desired.

If a carbohydrate source is included, it preferably comprises less than 10% by weight, preferably less than 5% by weight, more preferably less than 1% by weight of the composition. For some applications, such as e.g. ready-to-use beverages, compositions are advantageous which are essentially free from, or comprise less than 5% by weight of, mono-saccharides. If monosaccharides are present, glucose galactose and tagatose each preferably account for less than 40 % by weight, more preferably less than 10 % by weight, even more preferably less than 1 % by weight of the mono-saccharides. In other applications such as ready-to-use snacks, however, inclusion of a carbohydrate source may be advantageous, preferably in an amount to provide 1 to 70 %, more preferably 25 % to 45 % of the energy on basis of the total energy of the composition.

Non-caloric sweeteners, flavourings and food-acceptable colourings may also be included.

A particularly advantageous embodiment comprises a liquid composition such as a ready-to-use beverage based on fruit juice, vegetable juice, water, isotonic drinks, carbonated flavoured drinks, soft drinks, teas, coffees, dairy products, meat and/or vegetable soups or mixtures thereof, which may be supplemented with minerals, vitamins and/or carbonic acid, if desired.

Beverages comprising fruit or vegetable juices provide additionally the advantage of comprising vitamins, minerals or even enzymes and provide an advantageous complementation of a nutritional composition according to the present invention. In particular, juices such as orange, apple, pineapple, grapefruit, lemon, lime, mango, passion fruit, elderberries, cranberries, currants, grape, tomato, carrot or combinations thereof may form the basis for a ready-to-use beverage.

A liquid composition may comprise from 11 to 97 % by weight, preferably from 21 to 80 % by weight, most preferably from 61 to 75 % by weight, of any of the before-mentioned juices, beverages, water or mixtures thereof, and from 3 to 89 % by weight, preferably from 20 to 79 % by weight, most preferably from 25 to 39 % by weight, of a composition according to the present invention, on basis of the total weight of the fluid preparation.

Advantageously, a beverage according to the present invention delivers 1 to 150 kcal, preferably 21 to 100 kcal, more preferably 31 to 50 kcal per 100 g of liquid. For example, a beverage accompanying a standard meal may e.g. provide per dosage (i.e. per standard meal) 0.1 to 100 g, preferably 5 to 40 g acetogenic fibers, more preferably 10 to 30 g acetogenic fibers, even more preferably 20 g acetogenic fibers.

Of course, consumers may also prepare such a beverage by mixing a composition according to the present invention (e.g. according to instructions on the package) with a beverage of their choice.

Alternatively, a food product may be enriched with a composition according to the present invention. For example, a fermented milk, a yoghurt, a fresh cheese, a renneted milk, a confectionery bar, breakfast cereal flakes or bars, a drink, milk powder, soy-based product, non-milk fermented product or a nutritional supplement for clinical nutrition. Then, the amount of the composition added is preferably, at least 0.5 % by weight, more preferably 11 to 40 % by weight, on basis of the total weight of the food product.

Food products or beverages as detailed above, provide the advantage that they may be consumed shortly before, during, or shortly after a meal by a person and permit an easy solution for insulin sensitivity. Thus, compositions according to the present invention may be helpful in significantly increasing the quality of life of large groups of the population.

A composition according to the present invention may also be used for the preparation of an enteral nutritional formula, in particular for patients suffering from muscle depletion or for supporting muscle maintenance.

All before-mentioned products according to the present invention provide the advantage that they may be expected to be highly accepted by the consumers as they are formulated on basis of well-known nutritional components, which proved to be essentially free of undesired side-effects. Moreover, compositions according to the present invention are essentially free of unpleasant tastes and may be regularly, e.g. daily consumed.

The invention also provides a method for treating or preventing metabolic dysfunctions and/or improving insulin resistance (including Syndrome X) which comprises administering an effective amount of a composition according to the present invention.

The following examples are given by way of illustration only and should not be construed as limiting the subject-matter of the present application.

### Example 1

Eight obese and insulin resistant subjects received a primed constant intravenous infusion of [1-¹³C]acetate at the rate of 0.50 µmol.kg⁻¹.min⁻¹ and of [1,1,2,3,3-²H₅]glycerol at the rate of 0.11 µmol.kg⁻¹.min⁻¹ for 9 hours. After 3 hours of tracer infusion, patients ingested 30 g of pure lactulose, or saline solution. Arterialized blood samples were collected regularly.

Before saline or lactulose intake, plasma acetate turnover was similar; 11.4 ± 2.4 µmol.kg⁻¹. min⁻¹ with saline *vs* 10.7 ± 1.4 µmol.kg⁻¹.min⁻¹ with lactulose. Likewise, plasma glycerol turnover was 3.8 ± 0.4 µmol.kg⁻¹.min⁻¹ with saline *vs* 4.8 ± 1.9 µmol.kg⁻¹.min⁻¹ with lactulose. Plasma acetate concentrations were 201.1 ± 31.5 µmol/L and 221.5 ± 34.0 µmol/L respectively, plasma glycerol concentrations were 61.3 ± 10.9 µmol/L and 61.0 ± 8.8 µmol/L and FFA concentration were also stable. After lactulose ingestion, acetate turnover rate became significantly higher, 15.5 ± 2.2 µmol.kg⁻¹.min⁻¹ compared to 10.3 ± 2.2 µmol.kg⁻¹. min⁻¹ (*P* < 0.0001)with saline. Glycerol turnover decreased with lactulose ingestion compared to saline, 2.8 ± 0.4 *vs* 3.5 ± 0.3 µmol.kg⁻¹.min⁻¹ (*P* ≤ 0.05). A significant correlation was found between glycerol and acetate turnover post ingestion (r = - 0.78, *P* < 0.02). Acetate concentration increased to a maximum ≈ 400 µmol/L then decreased to baseline. FFA concentrations decreased significantly to 120 min thereafter increased slowly.

These results show that ingestion of lactulose and the associated increase in acetate production results in short-term changes in blood fatty acids indicating a decrease in lipolysis.

### Example 2

Twenty obese (body mass index between 25 and 35) male subjects are recruited for a randomised, single centre single blind clinical trial with a cross-over design to be carried out at Hospital Hotel Dieu, Nantes, France. The subjects are insulin resistant but have not developed full blown diabetes. The subjects are divided into two groups. Subjects in the first group receive twice daily 150ml of an aqueous solution of acetogenic fibres (20% apple pectin, 80% acacia gum) at a concentration of 100mg/ml (corresponding to a consumption of 30g of the fibre mixture per day). The solution is aromatised, sweetened and coloured. Subjects in the second group receive the same amount of aromatised, sweetened and coloured water. The solutions are administered between 8.30 and 10.30 in the morning and between 4 to 6 in the afternoon. The trial continues for 5 weeks.

The primary objective of the trial is to investigate the effect of a high intake of acetogenic fibres for five weeks on insulin sensitivity and the secondary objective is to monitor changes in insulinemia, glycemia, lipid parameters such as free fatty acids and glycerol plasma levels, cholesterol, phospholipids, triacylglycerides, glycerol, acetate and glucose kinetics, leptin and adiponectin and weight.

Before starting the trial, the insulin sensitivity of all subjects is assessed and a blood sample is taken for measurement of free fatty acids, insulin, glucose, glycerol, acetate, triacylglycerides, phospholipids, total cholesterol, HDL and LDL cholesterol, blood ionogram, creatinine, ALAT, ASAT leptin and adiponectin. Then the first group receive the solution containing acetogenic fibres for five weeks and the second group receive the placebo solution for five weeks. On the last day of the five week period, all subjects undergo a kinetic study and a euglycemic-hyperinsulinemic-clamp to determine changes in biochemistry and insulin sensitivity. After a period of six weeks, the insulin sensitivity of all subjects is again assessed using HOMA and the regime recommences with the first group receiving the placebo and the second group the acetogenic fibres for a further period of five weeks. On the last day of the five week period, all subjects undergo a kinetic study and a euglycemic-hyperinsulinemic-clamp. Subjects are advised to eat a normal diet throughout this sixteen week period.

In general it is found that the insulin sensitivity of the groups receiving the solution containing acetogenic fibres increases during the period of treatment also the plasma level of free fatty acids decreases and that these effects tend to persist for some time after the treatment has ceased.

It should be understood that various changes and modifications to the presently preferred embodiments described herein will be apparent to those skilled in the art. Such changes and modifications can be made without departing from scope of the present invention and without diminishing its attendant advantages.

## Claims

1. Use of a composition comprising acetogenic fibres for the preparation of a nutritional and/or a pharmaceutical composition for treating, preventing and/or improving insulin resistance.

2. The use according to claim 1, wherein the acetogenic fibre is lactulose, citrus pectin, apple pectin, carrot pectin, soybean fibre, soy fibre, acacia gum, gum Arabic or a mixture thereof.

3. The use according to claim 1 or 2, wherein the amount of acetogenic fibres in the composition is in the range or from 0.2 to 90 % by weight, preferably from 0.5 to 50 % by weight, more preferably 0.7 to 30 % by weight, even more preferably 5 to 25 % by weight, most preferred about 7 % by weight, based on the total weight of the composition.

## Patentansprüche

1. Verwendung einer Zusammensetzung, die acetogene Ballaststoffe umfasst, zur Herstellung einer Ernährungszusammensetzung und/oder einer pharmazeutischen Zusammensetzung zur Behandlung, Prävention und/oder Verbesserung der Insulinresistenz.

2. Verwendung nach Anspruch 1, wobei der acetogene Ballaststoff Lactulose, Citruspektin, Apfelpektin, Karottenpektin, Sojabohnenfaser, Sojafaser, Akaziengummi, Gummi arabicum oder eine Mischung daraus ist.

3. Verwendung nach Anspruch 1 oder 2, wobei die Menge an acetogenen Ballaststoffen in der Zusammensetzung im Bereich von 0,2 bis 90 Gew.-%, vorzugsweise von 0,5 bis 50 Gew.-%, stärker bevorzugt von 0,7 bis 30 Gew.-%, und noch stärker bevorzugt von 5 bis 25 Gew.-%, und am stärksten bevorzugt bei etwa 7 Gew.-%, bezogen auf das Gesamtgewicht der Zusammensetzung, liegt.

## Revendications

1. Utilisation d'une composition comprenant des fibres acétogènes pour la préparation d'une composition nutritionnelle et/ou pharmaceutique pour traiter, prévenir et/ou améliorer l'insulinorésistance.

2. Utilisation selon la revendication 1, dans laquelle la fibre acétogène est du lactulose, de la pectine d'agrumes, de la pectine de pomme, de la pectine de carotte, une fibre de graines de soja, une fibre de soja, de la gomme d'acacia, de la gomme arabique ou un mélange de ceux-ci.

3. Utilisation selon la revendication 1 ou 2, dans laquelle la quantité de fibres acétogènes dans la composition est comprise dans la plage de 0,2 à 90 % en poids, de préférence de 0,5 à 50 % en poids, de façon plus appréciée de 0,7 à 30 % en poids, de façon encore plus appréciée de 5 à 25 % en poids, de la façon la plus appréciée d'environ 7 % en poids, par rapport au poids total de la composition.
